# FASCICULE DE BREVET EUROPEEN

(11) **EP 1 779 844 B1**
(45) Date de publication et mention de la délivrance du brevet: **23.12.2009**
(21) Numéro de dépôt: 06291672.1
(22) Date de dépôt: 26.10.2006
(51) Int. Cl.: A61K 8/49, A61K 8/60, A61Q 5/00

(54) **Composition cosmétique comprenant au moins un agent antipelliculaire ainsi que du mono-laurate de sorbitan oxyéthyléné, et procédé de traitement cosmétique mettant en oeuvre ladite composition**
Kosmetische Zusammensetzung enthaltend eine Antischuppenverbindung und Oxyethylen-sorbitanmonolaurat
Cosmetic composition comprising an anti-dandruff compound and oxyethylenated sorbitan monolaurate

(30) Priorité: 28.10.2005 FR 0511095
(43) Date de publication de la demande: 02.05.2007
(73) Titulaire: L'Oréal, 75008 Paris (FR)
(72) Inventeur: Masse, Virginie, 92110 Clichy (FR); Decoster, Sandrine, 95210 Saint-Gratien (FR); Thomas, Béatrice, 92130 Issy Les Moulineaux (FR)
(74) Mandataire: Zapalowicz, Francis

(56) Documents cités:
- FR-A- 2 804 020
- US-A1- 2004 197 287
- DATABASE WPI Section Ch, Week 197942 Derwent Publications Ltd., London, GB; Class D21, AN 1979-76451B XP002391663 & JP 54 117037 A (SEIKEN KAGAKU KK) 11 septembre 1979 (1979-09-11)
- DATABASE WPI Section Ch, Week 199136 Derwent Publications Ltd., London, GB; Class A96, AN 1991-263819 XP002391664 & JP 03 173812 A (SUNSTAR KK) 29 juillet 1991 (1991-07-29)
- DATABASE WPI Section Ch, Week 198733 Derwent Publications Ltd., London, GB; Class A96, AN 1987-230385 XP002391665 & HU 42 318 A (INNOFINANCE ALTALANOS INNOVACI) 28 juillet 1987 (1987-07-28)

## Description

La présente invention est relative à une composition cosmétique comprenant au moins un agent antipelliculaire différent des dérivés de 2-pyridones, et au moins un mono-laurate de sorbitan oxyéthyléné comprenant de 2 à 25 unités oxyéthylène. La présente invention concerne également un procédé de traitement cosmétique des cheveux et du cuir chevelu mettant en oeuvre ladite composition cosmétique.

En vue de combattre la formation des pellicules, de nombreuses compositions anti-pelliculaires ont été proposées dans l'art antérieur.

Ainsi, la demande de brevet WO 2004/089318 décrit des compositions de shampooings présentant une action détergente et antipelliculaire améliorées. Ces compositions contiennent :
(a) de 5 à 50 % en poids d'un tensio-actif détergent,
(b) de 0,1 à 4 % en poids d'un agent anti-pelliculaire,
(c) de 0,1 à 50% en poids d'un dérivé polyoxyéthyléné d'un ester d'acide gras et de sorbitan, et
(d) au moins 20% en poids d'eau.

Le composé (c) est dérivé d'un ester de sorbitan et d'un acide gras en C14 à C18 tel que les acides palmitique, stéarique, oléique. Il correspond notamment à des produits tels que ceux vendus sous les noms TWEEN 40, TWEEN 60, TWEEN 61 et TWEEN 85 par la société UNIQEMA.

La demande de brevet EP 117135 décrit des compositions détergentes présentant des propriétés antimicrobiennes et antipelliculaires améliorées. Ces compositions comprennent au moins un tensioactif anionique ou amphotère, au moins un polymère azoté, et au moins une substance non particulaire soluble dans l'eau, telle que un agent antipelliculaire, un agent de protection solaire, un agent insecticide.

Les exemples présentent des compositions de shampooings antipelliculaires contenant, comme agent anti-pelliculaire, les produits dénommés OCTOPIROX ou OMADINE MDS. Certains de ces produits contiennent en outre du mono-laurate de sorbitan oxyéthylène à 40 OE, ou du mono-laurate de sorbitan oxyéthylène à 80 OE.

Enfin, la demande de brevet WO 03/096998 décrit des compositions de shampooings prévenant la formation des pellicules. Ces compositions contiennent comme agent actif des extraits de *Camelia sinensis,* ou leurs principes actifs du type des polyphénols (catéchines, flavonoïdes). Ces compositions peuvent également comprendre des agents antipelliculaires additionnels, et des agents tensioactifs anioniques, non ioniques amphotères, zwitterioniques et/ou cationiques. Parmi les agents tensio-actifs non ioniques, sont cités les esters d'acides gras et de sorbitan, et leurs dérivés polyoxyéthylénés comprenant de 1 à 30, et de préférence de 5 à 10 unités oxyéthylène.

Les compositions de l'art antérieur telles que celles décrites ci-avant ont toutes pour objectif une amélioration de l'effet purement antipelliculaire, et éventuellement une amélioration de la détergence. Par ailleurs, elles ne présentent pas toujours des propriétés d'usage satisfaisantes, notamment en terme de viscosité et de qualité de mousse.

Or, les utilisateurs de shampooings antipelliculaires présentent généralement des cuirs chevelus sensibles. De plus, l'usage fréquent de ce type de shampooings a tendance à accentuer les réactions d'inconfort telles que rougeurs, démangeaisons, picotements...

Ainsi, il existe un besoin de la part des utilisateurs de shampooings antipelliculaires de compositions présentant une meilleure tolérance cutanée, tout en conservant une bonne efficacité antipelliculaire et de bonnes propriétés d'usage.

La Demanderesse a maintenant découvert de manière surprenante qu'en associant à un agent antipelliculaire, différent des dérivés de 2-pyridones, un agent tensioactif particulier, il est possible de formuler une composition cosmétique qui présente une excellente activité antipelliculaire, tout en permettant de diminuer les phénomènes d'inconfort au niveau du cuir chevelu et ayant de bonnes propriétés d'usage notamment en terme de viscosité.

Ainsi, les compositions selon l'invention, qui comprennent au moins un agent antipelliculaire différent des dérivés de 2-pyridones associé à au moins un mono-laurate de sorbitan oxyéthyléné comprenant de 2 à 10 unités oxyéthylène, présentent une excellente tolérance cutanée.

Par ailleurs, en plus de leur très bonne efficacité antipelliculaire, les compositions selon l'invention présentent de très bonnes propriétés tant nettoyantes que cosmétiques.

La présente invention a donc pour objet une composition cosmétique comprenant, dans un milieu aqueux, au moins un agent antipelliculaire différent des dérivés de 2-pyridones, et au moins un mono-laurate de sorbitan oxyéthyléné comprenant de 2 à 10 unités oxyéthylène.

Un autre objet de l'invention est un procédé de traitement cosmétique mettant en oeuvre ladite composition.

D'autres objets et caractéristiques, aspects et avantages de l'invention apparaîtront encore plus clairement à la lecture de la description et des exemples qui suivent.

Selon l'invention, la composition cosmétique comprend dans un milieu aqueux, au moins un agent antipelliculaire différent des dérivés de 2-pyridones, et au moins un mono-laurate de sorbitan oxyéthyléné comprenant de 2 à 10 unités oxyéthylène.

Dans le présent exposé, et de manière bien connue en soi, on désigne par composé oxyéthyléné à X OE un composé oxyéthyléné comprenant X unités oxyéthylène par molécule.

Les agents antipelliculaires, utilisables dans la composition objet de la présente invention, sont des composés connus de l'homme du métier. De nombreux composés peuvent ainsi être employés à cet effet, à l'exclusion des dérivés de 2-pyridones.

Lesdits agents antipelliculaires peuvent être tout agent actif utile pour prévenir l'apparition des pellicules, diminuer leur nombre et/ou les faire disparaître totalement. Ainsi, l'agent antipelliculaire peut être choisi parmi les agents antifongiques et/ou antibactériens.

Les agents antipelliculaires utilisables selon l'invention peuvent être notamment choisis parmi :
1) les sels de pyridinethione notamment les sels de calcium, de magnésium, de barium, de strontium, de zinc, de cadmium, d'étain et de zirconium. Le sel de zinc de pyridinethione est particulièrement préféré. Le sel de zinc de pyridinethione est notamment commercialisé sous la dénomination Omadine de zinc par la société OLIN.
2) les trihalogéno carbamide de formule: 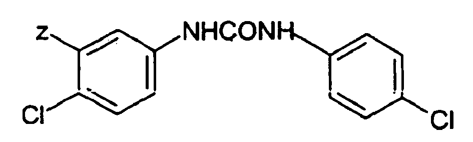 dans laquelle Z représente un atome d'halogène comme le chlore ou un groupement trihalogénoalkyle en C₁-C₄ tel que CF₃.
3) le triclosan représenté par la formule : 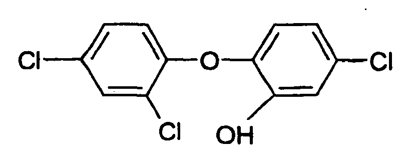
4) les composés azolés tels que le climbazole, le kétoconazole, le clotrinazole, l'econazole, l'isoconazole et le miconazole.
5) les polymères antifongiques tels que l'amphotéricine B ou la nystatine.
6) les sulfures de sélénium en particulier ceux de formule SₓSe₈₋ₓ , x allant de 1 à 7.
7) d'autres agents antipelliculaires, sont le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.

A titre d'exemples préférentiels d'agents antipelliculaires, on peut notamment citer la pyrithione de zinc, l'acide salicylique, le disulfure de sélénium, et leurs mélanges.

La composition selon l'invention comprend avantageusement de 0,001 à 10 % en poids d'agent(s) antipelliculaire(s), de préférence de 0,1 à 5 % en poids, encore plus préférentiellement de 0,2 à 2 % en poids, par rapport au poids total de la composition.

La composition objet de la présente invention comprend un agent tensio-actif particulier, constitué d'au moins un mono-laurate de sorbitan oxyéthyléné comprenant de 2 à 10 unités oxyéthylène.

De manière encore plus préférée, la composition selon l'invention comprend du mono-laurate de sorbitan oxyéthylène à 4 OE. Ce composé est également connu sous le nom de polysorbate 21. Il est, entre autres, commercialisé sous la dénomination TWEEN 21 par la société UNIQEMA.

Ce composé peut être utilisé seul, ou en mélange avec d'autres dérivés oxyéthylénés, notamment ceux du mono-laurate de sorbitan. Par exemple, il est possible d'employer dans le cadre de la présente invention du mono-laurate de sorbitan oxyéthylène à 4 OE, en mélange avec du mono-laurate de sorbitan oxyéthylène à 20 OE.

La composition selon l'invention comprend avantageusement au moins 0,5 % en poids de mono-laurate de sorbitan oxyéthyléné comprenant de 2 à 10 unités oxyéthylène, par rapport au poids total de la composition. De préférence, elle comprend de 0,5 à 10 % en poids dudit mono-laurate de sorbitan oxyéthylèné, plus préférentiellement de 2 à 9 % en poids, encore plus préférentiellement de 3 à 8 % en poids, par rapport au poids total de la composition.

La composition selon l'invention peut également comprendre un ou plusieurs alcool gras comportant au moins 12 atomes de carbone, de préférence de 12 à 30 atomes de carbone et encore plus préférentiellement de 16 à 24 atomes de carbone.

A titre d'exemples non limitatifs de tels alcools gras, on peut notamment citer les alcools cétylique, stéarylique, arachidylique, béhénylique, lignocérylique, cérylique et montanylique, et leurs mélanges, et plus particulièrement l'alcool béhénylique.

L'alcool gras est présent de préférence en une proportion supérieure ou égale à 0,5 % en poids, allant de préférence de 0.5% à 10 % en poids et mieux encore de 1 à 5 % en poids, par rapport au poids total de la composition.

Les compositions selon l'invention peuvent se présenter sous la forme de shampooings, de compositions à appliquer avant ou après un shampooing, ces dernières se présentant sous la forme d'une lotion plus ou moins épaissie, d'un gel ou d'une émulsion,

Les compositions de l'invention peuvent également comprendre un ou plusieurs tensioactifs choisis parmi les tensioactifs anioniques, cationiques, amphotères et non ioniques différents du monolaurate de sorbitane, oxytéthyléné comprenant de 2 à 10 unités oxyéthylène.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,1 à 50 % en poids, mieux encore de 4 à 20% en poids par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels est de préférence comprise dans l'intervalle allant de 0,01 à 20 % en poids, mieux encore de 0,2 à 10 % en poids par rapport au poids total de la composition.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (II) et (III) :

Rₐ-CONHCH₂CH₂-N(R_{b})(R_{c})(CH₂COO⁻) (II)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ'-CONHCH₂CH₂-N(B)(B') (III)
dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,01 à 20 % en poids, mieux encore de 0,5 à 10% en poids par rapport au poids total de la composition.

De manière avantageuse, la composition selon l'invention comprend au moins un tensioactif anionique et au moins un tensioactif amphotère ou zwittérionique.

La composition selon l'invention présente de préférence une teneur totale en tensioactifs anioniques, non-ioniques, amphotères et zwittérioniques comprise dans l'intervalle allant de 4 à 50 % en poids, mieux encore de 4 à 20 % en poids, par rapport au poids total de la composition.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Lorsque les tensioactifs cationiques sont présents, leur quantité est de préférence comprise dans l'intervalle allant de 0,01 à 10 % en poids, mieux encore de 0,2 à 5 % en poids, et encore plus préférentiellement de 0,3 à 3 % en poids par rapport au poids total de la composition cosmétique.

Les compositions selon la présente invention peuvent comprendre en outre au moins un polymère cationique.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, de préférence supérieure à 10⁶ et mieux encore comprise entre 10⁶ et 10⁸.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides acryliques ou méthacryliques et comportant au moins un des motifs de formules suivantes: 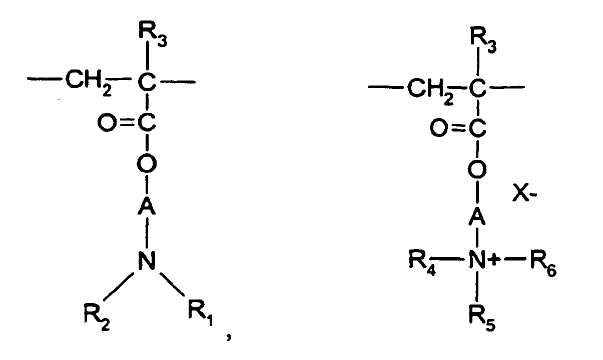 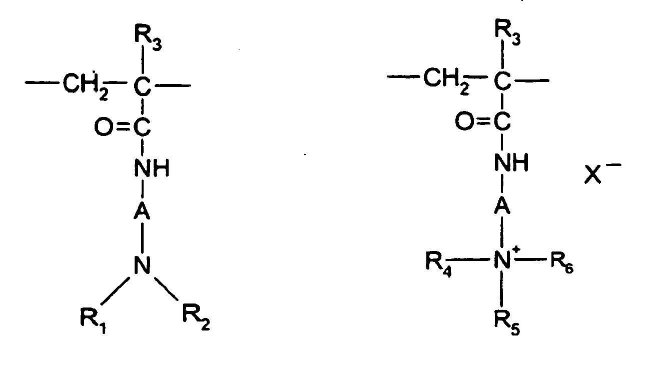 dans lesquelles:
   R₁ et R₂, identiques ou différents, représentent un atome d'hydrogène ou un groupe alkyle ayant de 1 à 6 atomes de carbone, et de préférence un groupe méthyle ou éthyle ;
   R₃, identiques ou différents, désignent un atome d'hydrogène ou un groupe CH₃ ;
   les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone ;
   X désigne un anion dérivé d'un acide minéral ou organique tel qu'un anion méthosulfate ou un halogénure comme le chlorure ou le bromure.

   Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle,
   - les copolymères d'acrylamide et de chlorure de méthacryloyloxyéthyltriméthylammonium décrits, par exemple, dans la demande de brevet EP-A-080976,
   - les copolymères d'acrylamide et de méthosulfate de méthacryloyloxyéthyltriméthylammonium,
   - les copolymères vinylpyrrolidone/acrylate ou méthacrylate de dialkylaminoalkyle quaternisés ou non. Ces polymères sont décrits en détail dans les brevets français 2 077 143 et 2 393 573,
   - les terpolymères méthacrylate de diméthylaminoéthyle/vinylcaprolactame/vinylpyrrolidone,
   - les copolymères vinylpyrrolidone/méthacrylamidopropyl-diméthylamine, et
   - les copolymères vinylpyrrolidone/méthacrylamide de diméthylaminopropyle quaternisé.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination "Celquat^{®} L 200" et "Celquat^{®} H 100" par la Société National Starch.
(4) Les polysaccharides cationiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C13 S, JAGUAR^{®} C15, JAGUAR^{®} C17 ou JAGUAR^{®} C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
   Parmi ces dérivés, on peut citer plus particulièrement les polymères acide adipique/diméthylaminohydroxypropyl-diéthylène-triamine.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 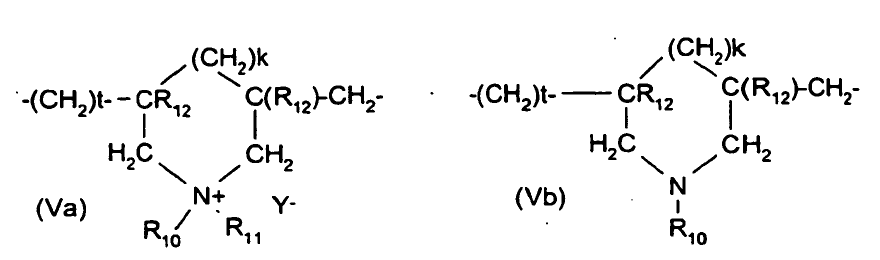 dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT® 100" par la société CALGON (et ses homologues de faibles masses moléculaires moyenne en poids) et les copolymères de chlorure de diallyldiméthylammonium et d'acrylamide commercialisés sous la dénomination "MERQUAT^{®} 550".
(10) Les polymères de diammonium quaternaire contenant des motifs récurrents répondant à la formule (VI) : 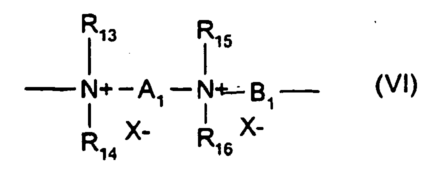 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)ₙ-

      dans lequel E' désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH-.

   De préférence, X- est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule : 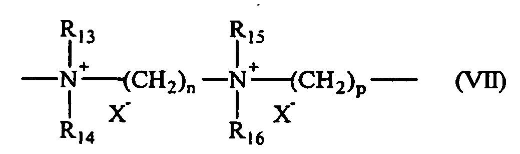 dans laquelle R₁₃, R₁₄, R₁₅ et R₁₆, identiques ou différents, désignent un groupe alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs de formule (VIII) : 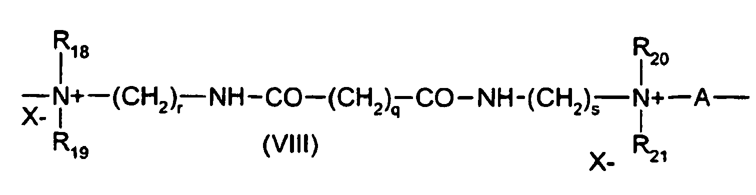 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogénure,
   A désigne un radical d'un dihalogénure ou représente de préférence -CH₂-CH₂-O-CH₂-CH₂-.

   De tels composés sont notamment décrits dans la demande de brevet EP-A-122 324.
(12) Les polymères quaternaires de vinylpyrrolidone et de vinylimidazole.
(13) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société UNION CARBIDE CORPORATION, les cyclopolymères cationiques, en particulier les homopolymères ou copolymères de chlorure de diméthyldiallylammonium, vendus sous les dénominations MERQUAT® 100, MERQUAT® 550 et MERQUAT® S par la société CALGON, les gommes de guar modifiées par un sel de 2,3-époxypropyl-triméthylammonium, les polymères quaternaires de vinylpyrrolidone et de vinylimidazole.

Lorsque les polymères cationiques sont présents, ils sont de préférence présents en une quantité allant de 0,01 à 10 % en poids, mieux encore de 0,02 à 5 % en poids, et encore plus préférentiellement de 0,05 à 1 % en poids par rapport au poids total de la composition.

La composition selon l'invention peut comprendre en outre au moins une silicone.

Les silicones utilisables conformément à l'invention peuvent être solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Ils peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique: 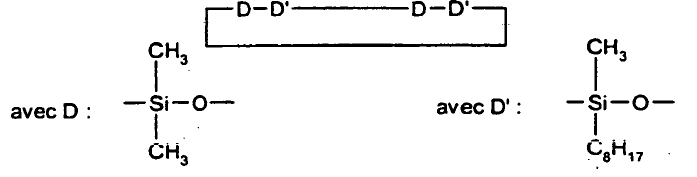 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 110⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphényl-siloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Peuvent également être employés des mélanges de silicones tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements ammonium quaternaires comme les produits commercialisés sous les dénominations ABILQUAT 3272 et ABILQUAT 3474 par la société GOLDSCHMIDT ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes tels que les polydiméthylsiloxanes à groupements terminaux triméthylsilyle, ou les polydiméthylsiloxanes à groupements terminaux hydroxydiméthylsilyle, et les silicones aminées.

Lorsque lesdites silicones sont présentes, elles sont contenues de préférence en une quantité allant de 0,05 à 20 % en poids, plus particulièrement de 0,1 à 10 % en poids, et mieux encore de 0,5 à 5 % en poids par rapport au poids total de la composition.

Le milieu aqueux est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention est généralement inférieur à 8,5, et de préférence compris dans l'intervalle allant de 4 à 7.

La composition selon l'invention peut comprendre en outre un ou plusieurs additifs classiques bien connus dans la technique, tels que les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

Comme agents opacifiants, peuvent être employés par exemple des composés tels que l'hydrostéaryl cétyl éther, le distéarate d'éthylène glycol.

La composition selon l'invention peut ainsi comprendre un ou plusieurs éthers à deux chaînes grasses répondant à la formule suivante:

R-O-R' (I)

dans laquelle :
R et R', identiques ou différents, désignent un groupe alkyle ou alcényle, linéaire ou ramifié, comportant de 12 à 30 atomes de carbone et de préférence de 14 à 24 atomes de carbone, R et R' étant choisis de façon telle que le composé de formule (I) soit solide à une température égale à 30° C environ. De préférence, R et R' sont identiques et désignent un groupe alkyle tel que stéaryle.

En particulier, l'éther à deux chaînes grasses peut être le distéaryléther, tel que par exemple le produit vendu sous la dénomination CUTINA®STE par la société COGNIS.

L'éther à deux chaînes grasses est présent de préférence en une proportion supérieure ou égale à 0,5 % en poids, mieux encore de 1 à 5 % en poids, et encore plus préférentiellement de 1,3 à 2 % en poids, par rapport au poids total de la composition.

L'homme de métier veillera à choisir les éventuels additifs et leurs quantités de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

La composition selon l'invention peut être utilisée notamment comme composition de traitement ou de soin cosmétique des cheveux. En particulier, elle peut être utilisée comme shampooing ou composition à appliquer avant ou après un shampooing.

Un autre objet de l'invention est un procédé de traitement cosmétique des cheveux, qui comprend l'application sur les cheveux d'une quantité efficace d'une composition cosmétique telle que décrite ci-dessus.

Selon un mode de mise en oeuvre préféré, un tel procédé consiste à appliquer sur les cheveux une quantité efficace de la composition cosmétique, à éventuellement rincer après un éventuel temps de pause.

Lorsque l'on applique la composition selon l'invention sous forme d'une lotion ou d'une crème avant ou après shampooing, on la laisse éventuellement pauser sur les cheveux pendant environ 1/2 minute à 5 minutes, puis on rince éventuellement à l'eau.

Les exemples suivants sont donnés à titre illustratif de la présente invention, et ne sauraient en limiter la portée.

Dans les exemples suivants, toutes les quantités sont indiquées en pour cent en poids de matière active par rapport au poids total de la composition, sauf indication contraire.

### EXEMPLES

### Exemples 1 à 3 :

Trois compositions de shampooing, conformes à l'invention, sont préparées à partir des ingrédients indiqués dans le tableau ci-dessous.

| Compositions | 1 | 2 | 3 |
|---|---|---|---|
| lauryl éther sulfate de sodium à 2,2 OE en solution aqueuse (Texapon N702 de Cognis) | 15,6 | 11,7 | 15,6 |
| cocoyl amidopropyl bétaine / monolaurate de glycérine en solution aqueuse 25 / 75 (Tégobétaine HS de Goldschmidt) | 2,5 | 1,8 | 2,4 |
| mono-laurate de sorbitan oxyéthylène à 4 OE (Tween 21 de Uniqema) | 4 | 4 | 4 |
| mélange de 1-(hexadécyloxy)-2-octadécanol / alcool cétylique 47/53 | 2,5 | 2,5 | 2,5 |
| pyrithione de zinc en dispersion aqueuse (Zinc omadine pyrithione 48% de Arch Personal Care) | 1 | 1 | 1 |
| poly diméthylsiloxane (PM : 250.000) (Mirasil DM 500 000 de Rhodia) | 1,5 | 1 | 2,7 |
| hexylène glycol (2 méthyl-2,4 pentanediol) | 0,8 | 0,5 | |
| chlorure de sodium | 0,6 | | |
| parfum | 0,5 | 0,5 | 0,5 |
| hydroxyéthyl cellulose quaternisée par du chlorure de 2,3 époxypropyl triméthyl ammonium (Celquat SC 240C de National Starch) | 0,4 | 0,4 | |
| monoisopropanolamide d'acides de coprah (Empilan cis de Huntsman) | 0,35 | 1,2 | 0,5 |
| polymère carboxyvinylique synthétisé dans un mélange acétate d'éthyle / cyclohexane (Carbopol 980 de Noveon) | 0,3 | 0,3 | 0,3 |
| diméthylol-1,3 diméthyl-5,5 hydantoine en solution aqueuse (Glydant LTD de Lonza) | 0,14 | 0,14 | 0,14 |
| p-hydroxybenzoate de méthyle, sel de sodium (Nipagin M sodium de Clariant) | 0,2 | 0,2 | 0,2 |
| propylène glycol | 0,1 | 0,1 | 0,1 |
| mélange p-hydroxybenzoates de méthyle, éthyle, propyle, butyle, isobutyle, phénoxy-2-éthanol (Phenonip de Clariant) | 0,05 | 0,05 | 0,05 |
| chlorure d'hydroxypropyl guar triméthylammonium (Jaguar C 13 S de Rhodia) | | | 0,05 |
| Eau qsp | 100% | 100% | 100% |

Les compositions 1, 2 et 3 conformes à l'invention se sont avérées présenter une excellente tolérance vis-à-vis du cuir chevelu. En particulier, l'on a observé une très nette diminution des réactions d'inconfort.

En outre, ces compositions présentent une efficacité antipelliculaire d'un très bon niveau, au moins identique, voire supérieure aux compositions classiques ne contenant pas de mono-laurate de sorbitan oxyéthylène à 4 OE.

Enfin, ces compositions présentent de remarquables propriétés cosmétiques. En particulier, elles présentent une consistance très agréable, elles sont très faciles à répartir et à rincer.

Après application sur les cheveux de l'une ou l'autre de ces compositions, puis rinçage, les cheveux humides sont propres et agréables au toucher. Une fois séchés, les cheveux sont faciles à mettre en forme, et présentent une douceur et un toucher très agréable. Ils sont brillants et souples.

### Exemples 4 à 6 :

Trois compositions de shampooings antipelliculaires sont préparées à partir des ingrédients indiqués dans le tableau ci-dessous. La composition 4 est conforme à l'invention, les compositions 5 et 6 sont présentées à titre comparatif.

| Compositions | 4 | 5 | 6 |
|---|---|---|---|
| lauryl éther sulfate de sodium (2,2 OE) en solution aqueuse (Texapon N702 de Cognis) | 15,4 | 15,4 | 15,4 |
| cocoyl amidopropyl bétaïne / monolaurate de glycérine en solution aqueuse 25 / 75 (Tégobétaïne de Goldschmidt) | 1,7 | 1,7 | 1,7 |
| mono-laurate de sorbitan oxyéthylène à 4 OE (Tween 21 de Uniqema) | 6 | - | - |
| mono-palmitate de sorbitan oxyéthylène à 20 OE (Tween 40 de Uniqema) | - | - | 6 |
| mono-stéarate de sorbitan oxyéthylène à 4 OE (Tween 61 de Uniqema) | - | 6 | - |
| distéarate d'éthylène glycol (Tegin BL 315 de Goldschmidt) | 2 | 2 | 2 |
| N-cocoyl amidoéthyl, N-éthoxycarboxyméthyl glycinate de sodium (Miranol C2M conc de Rhodia) | 0,8 | 0,8 | 0,8 |
| poly diméthylsiloxane (Mirasil DM 500 000 de Rhodia) | 1,5 | 1,5 | 1,5 |
| monoisopropanolamide d'acides de coprah (Empilan cis de Huntsman) | 0,3 | 0,3 | 0,3 |
| pyrithione de zinc en dispersion aqueuse (Zinc omadine pyrithione 48% de Arch Personal Care) | 1 | 1 | 1 |
| benzoate de sodium | 0,5 | 0,5 | 0,5 |
| parfum | 0,5 | 0,5 | 0,5 |
| mélange de p-hydroxybenzoates de méthyle, butyle, éthyle, propyle, isobutyle (7/57/22/14) (Nipastat de Nipa) | 0,5 | 0,5 | 0,5 |
| hydroxyéthyl cellulose quaternisée par du chlorure de 2,3 époxypropyl triméthyl ammonium (Celquat SC 240C de National Starch) | 0,4 | 0,4 | 0,4 |
| acide salicylique en poudre | 0,2 | 0,2 | 0,2 |
| polymère carboxyvinylique synthétisé dans un mélange acétate d'éthyle / cyclohexane (Carbopol 980 de Noveon) | 0,2 | 0,2 | 0,2 |
| p-hydroxybenzoate de méthyle, sel de sodium (Nipagin M sodium de Clariant) | 0,03 | 0,03 | 0,03 |
| Eau qsp | 100% | 100% | 100% |

La composition 4 selon l'invention présente en particulier, par rapport aux compositions comparatives 5 et 6, des avantages en termes de mise en oeuvre, et de viscosité.

En premier lieu, le mono-stéarate de sorbitan oxyéthylène à 4 OE employé dans la composition comparative 5 doit être fondu avant son incorporation dans le shampooing, ce qui rend la préparation de ce dernier plus difficile. A l'inverse, le mono-laurate de sorbitan oxyéthylène à 4 OE employé dans la composition 4 conforme à l'invention est liquide à température ambiante. Il peut donc être incorporé dans le shampooing à température ambiante, d'où un gain substantiel en termes de temps et d'énergie.

En second lieu les viscosités des compositions comparatives 5 et 6 ne sont pas adéquates, par rapport à la consistance habituelle des shampooings.

En effet, la composition 5 présente un temps d'écoulement à 25°C de 229 s CF 10 (Coupe Ford N° 10), ce qui correspond à une viscosité trop élevée (écoulement discontinu). A l'inverse, la composition 6 présente un temps d'écoulement à 25°C de 5 s CF 10, ce qui correspond à une viscosité beaucoup trop basse.

La composition 4 selon l'invention présente un temps d'écoulement à 25°C de 112 s CF 10, ce qui correspond à un écoulement continu et représente une viscosité plus appropriée pour un shampooing.

Ainsi, la composition 4 conforme à l'invention présente des qualités d'usage supérieures aux compositions comparatives : elle n'est ni trop épaisse, ni trop liquide, permettant ainsi une meilleure répartition du produit sur les cheveux.

## Revendications

1. Composition cosmétique comprenant, dans un milieu aqueux, au moins un agent antipelliculaire différent des dérivés de 2-pyridones, et au moins un mono-laurate de sorbitan oxyéthyléné comprenant de 2 à 10 unités oxyéthylène.

2. Composition selon la revendication précédente, **caractérisée en ce que** l'agent antipelliculaire est choisi parmi :
a) les sels de pyridinethione notamment les sels de calcium, de magnésium, de barium, de strontium, de zinc, de cadmium, d'étain et de zirconium,
b) les trihalogéno carbamides,
c) le triclosan,
d) les composés azolés tels que le climbazole, le kétoconazole, le clotrinazole, l'econazole, l'isoconazole et le miconazole,
e) les polymères antifongiques tels que l'amphotéricine B ou la nystatine,
f) les sulfures de sélénium en particulier ceux de formule SₓSe₈₋ₓ , x allant de 1 à 7,
g) le soufre sous ses différentes formes, le sulfure de cadmium, l'allantoïne, les goudrons de houille ou de bois et leurs dérivés, l'acide salicylique, l'acide undécylénique, l'acide fumarique, les allylamines telle que la terbinafine.

3. Composition selon la revendication précédente, **caractérisée en ce que** l'agent antipelliculaire est choisi parmi la pyrithione de zinc, l'acide salicylique, le disulfure de sélénium, et leurs mélanges.

4. Composition selon l'une quelconque des revendications précédentes, **caractérisée en ce qu'**elle comprend de 0,001 à 10 % en poids d'agent(s) antipelliculaire(s), par rapport au poids total de la composition.

5. Composition selon la revendication précédente, **caractérisée en ce qu'**elle comprend de 0,1 à 5 % en poids, de préférence de 0,2 à 2 % en poids d'agent(s) antipelliculaire(s), par rapport au poids total de la composition.

6. Composition selon la revendication 1, **caractérisée en ce qu'**elle comprend du mono-laurate de sorbitan oxyéthylène à 4 OE.

7. Composition selon l'une quelconque des revendications précédentes, **caractérisée en ce qu'**elle comprend au moins 0,5 % en poids de mono-laurate de sorbitan oxyéthyléné comprenant de 2 à 10 unités oxyéthylène, par rapport au poids total de la composition.

8. Composition selon la revendication précédente, **caractérisée en ce qu'**elle comprend de 0,5 à 10 % en poids dudit mono-laurate de sorbitan oxyéthylèné, de préférence de 2 à 9 % en poids, par rapport au poids total de la composition.

9. Composition selon l'une quelconque des revendications précédentes, **caractérisée en ce qu'**elle comprend également au moins un éther à deux chaînes grasses choisi parmi les éthers à deux chaînes grasses, solides à une température égale à environ 30°C, répondant à la formule suivante :
R-O-R' (I)
dans laquelle :
R et R', identiques ou différents, désignent un groupe alkyle ou alcényle, linéaire ou ramifié, comportant de 12 à 30 atomes de carbone, R et R' étant choisis de façon telle que le composé de formule (I) soit solide à une température égale à 30° C environ.

10. Composition selon la revendication précédente, **caractérisée en ce que** l'éther à deux chaînes grasses est le distéaryléther.

11. Composition selon l'une quelconque des revendications 9 et 10, **caractérisée en ce que** l'éther à deux chaînes grasses est présent en une quantité supérieure ou égale à 0,5 % en poids par rapport au poids total de la composition.

12. Composition selon la revendication 11, **caractérisée en ce que** l'éther à deux chaînes grasses est contenu en une quantité allant de 1 à 5 % en poids, de préférence de 1,3 à 2 % en poids, par rapport au poids total de la composition.

13. Composition selon l'une quelconque des revendications précédentes, **caractérisée en ce qu'**elle comprend en outre au moins un alcool gras comportant au moins 12 atomes de carbone.

14. Composition selon la revendication 13, **caractérisée en ce que** l'alcool gras comporte de 12 à 30 atomes de carbone.

15. Composition selon la revendication 13 ou 14, **caractérisée en ce que** l'alcool gras est choisi parmi les alcools cétylique, stéarylique, arachidylique, béhénylique, lignocérylique, céryliqué et nontanylique, et leurs mélanges.

16. Composition selon l'une quelconque des revendications 13 à 15, **caractérisée en ce que** l'alcool gras est présent en une quantité supérieure ou égale à 0,5 % en poids par rapport au poids total de la composition.

17. Composition selon la revendication 16, **caractérisée en ce que** l'alcool gras est présent en une quantité allant de 0,5 à 10 % et de préférence de 1 à 5 % en poids par rapport au poids total de la composition.

18. Composition selon l'une quelconque des revendications précédentes, **caractérisée en ce qu'**elle contient en outre au moins un tensioactif anionique, cationique, amphotère ou non ionique différent du monolaurate de sorbitan oxyéthyléné comprenant de 2 à 10 unités oxythylène.

19. Composition selon la revendication 18, **caractérisée en ce qu'**elle comprend au moins un tensioactif anionique et au moins un tensioactif amphotère ou zwittérionique.

20. Composition selon la revendication 18 ou 19, **caractérisée en ce que** le tensioactif anionique est choisi parmi les alkylsulfates, les alkyléthersulfates et leurs mélanges.

21. Composition selon l'une quelconque des revendications 18 à 20, **caractérisée en ce qu'**elle contient de 0,1 à 50 % en poids de tensioactif(s) anionique(s), de préférence de 4 à 20% en poids, par rapport au poids total de la composition.

22. Composition selon l'une quelconque des revendications 18 ou 19, **caractérisée en ce que** le tensioactif amphotère ou zwittérionique est choisi parmi les (alkyl en C₈₋₂₀)-bétaïnes, les (alkyl en C₈₋₂₀)-amido(alkyl en C₆₋₈)bétaïnes et leurs mélanges.

23. Composition selon l'une quelconque des revendications précédentes, **caractérisée en ce qu'**elle comprend en outre au moins un polymère cationique.

24. Composition selon l'une quelconque des revendications précédentes, **caractérisée en ce qu'**elle comprend en outre au moins une silicone.

25. Composition selon l'une quelconque des revendications précédentes, **caractérisée en ce que le** milieu aqueux est constitué d'eau ou d'un mélange d'eau et d'au moins un solvant cosmétiquement acceptable.

26. Composition selon la revendication 25, **caractérisée en ce que** le solvant est choisi parmi les alcools inférieurs en C₁-C₄ et les polyols.

27. Composition selon l'une quelconque des revendications précédentes, **caractérisée en ce qu'**elle comprend en outre au moins un additif choisi parmi les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

28. Utilisation de la composition selon l'une quelconque des revendications 1 à 27, comme composition de traitement ou de soin cosmétique des cheveux.

29. Utilisation de la composition selon l'une quelconque des revendications 1 à 27, comme shampooing ou composition à appliquer avant ou après un shampooing.

30. Procédé de traitement cosmétique, **caractérisé en ce qu'**il comprend l'application sur les cheveux d'une quantité efficace d'une composition selon l'une quelconque des revendications 1 à 27.

## Claims

1. Cosmetic composition comprising, in an aqueous medium, at least one anti-dandruff agent other than 2-pyridone derivatives, and at least one oxyethylenated sorbitan monolaurate comprising from 2 to 10 oxyethylene units.

2. Composition according to the preceding claim, **characterized in that** the anti-dandruff agent is chosen from:
a) pyridinethione salts, in particular the calcium, magnesium, barium, strontium, zinc, cadmium, tin and zirconium salts,
b) trihalocarbamides,
c) triclosan,
d) azole compounds such as climbazole, ketoconazole, clotrinazole, econazole, isoconazole and miconazole,
e) antifungal polymers such as amphotericin B or nystatin,
f) selenium sulphides, in particular those of formula SₓSe₈₋ₓ, x ranging from 1 to 7,
g) sulphur in its various forms, cadmium sulphide, allantoin, coal tar or wood tar and derivatives thereof, salicylic acid, undecylenic acid, fumaric acid, allylamines such as terbinafine.

3. Composition according to the preceding claim, **characterized in that** the anti-dandruff agent is chosen from zinc pyrithione, salicylic acid and selenium disulphide, and mixtures thereof.

4. Composition according to any one of the preceding claims, **characterized in that** it comprises from 0.001 to 10% by weight of anti-dandruff agent(s), relative to the total weight of the composition.

5. Composition according to the preceding claim, **characterized in that** it comprises from 0.1 to 5% by weight, preferably from 0.2 to 2% by weight of anti-dandruff agent(s), relative to the total weight of the composition.

6. Composition according to Claim 1, **characterized in that** it comprises oxyethylene sorbitan monolaurate comprising 4 OE.

7. Composition according to any one of the preceding claims, **characterized in that** it comprises at least 0.5% by weight of oxyethylenated sorbitan monolaurate comprising from 2 to 10 oxyethylene units, relative to the total weight of the composition.

8. Composition according to the preceding claim, **characterized in that** it comprises from 0.5 to 10% by weight of said oxyethylenated sorbitan monolaurate, preferably from 2 to 9% by weight, relative to the total weight of the composition.

9. Composition according to any one of the preceding claims, **characterized in that** it also comprises at least one ether having two fatty chains, chosen from ethers having two fatty chains, which are solid at a temperature equal to approximately 30°C, corresponding to the following formula:
R-O-R' (I)
in which:
R and R', which may be identical or different, denote a linear or branched alkyl or alkenyl group containing from 12 to 30 carbon atoms, R and R' being chosen in such a way that the compound of formula (I) is solid at a temperature equal to approximately 30°C.

10. Composition according to the preceding claim, **characterized in that** the ether having two fatty chains is distearyl ether.

11. Composition according to either one of Claims 9 and 10, **characterized in that** the ether having two fatty chains is present in an amount greater than or equal to 0.5% by weight relative to the total weight of the composition.

12. Composition according to Claim 11, **characterized in that** the ether having two fatty chains is contained in an amount ranging from 1 to 5% by weight, preferably from 1.3 to 2% by weight, relative to the total weight of the composition.

13. Composition according to any one of the preceding claims, **characterized in that** it also comprises at least one fatty alcohol containing at least 12 carbon atoms.

14. Composition according to Claim 13, **characterized in that** the fatty alcohol contains from 12 to 30 carbon atoms.

15. Composition according to Claim 13 or 14, **characterized in that** the fatty alcohol is chosen from cetyl alcohol, stearyl alcohol, arachidyl alcohol, behenyl alcohol, lignoceryl alcohol, ceryl alcohol and montanyl alcohol, and mixtures thereof.

16. Composition according to any one of Claims 13 to 15, **characterized in that** the fatty alcohol is present in an amount greater than or equal to 0.5% by weight relative to the total weight of the composition.

17. Composition according to Claim 16, **characterized in that** the fatty alcohol is present in an amount ranging from 0.5 to 10%, and preferably from 1 to 5% by weight relative to the total weight of the composition.

18. Composition according to any one of the preceding claims, **characterized in that** it also comprises at least one anionic, cationic, amphoteric or nonionic surfactant different from the oxyethylenated sorbitan monolaurate comprising from 2 to 10 oxyethylene units.

19. Composition according to Claim 18, **characterized in that** it comprises at least one anionic surfactant and at least one amphoteric or zwitterionic surfactant.

20. Composition according to Claim 18 or 19, **characterized in that** the anionic surfactant is chosen from alkyl sulphates and alkyl ether sulphates, and mixtures thereof.

21. Composition according to any one of Claims 18 to 20, **characterized in that** it contains from 0.1 to 50% by weight of anionic surfactant(s), preferably from 4 to 20% by weight, relative to the total weight of the composition.

22. Composition according to either one of Claims 18 and 19, **characterized in that** the amphoteric or zwitterionic surfactant is chosen from (C₈₋₂₀ alkyl)betaines and (C₈₋₂₀ alkyl)amino(C₆₋₈ alkyl)betaines, and mixtures thereof.

23. Composition according to any one of the preceding claims, **characterized in that** it also comprises at least one cationic polymer.

24. Composition according to any one of the preceding claims, **characterized in that** it also comprises at least one silicone.

25. Composition according to any one of the preceding claims, **characterized in that** the aqueous medium consists of water or of a mixture of water and of at least one cosmetically acceptable solvent.

26. Composition according to Claim 25, **characterized in that** the solvent is chosen from C₁-C₄ lower alcohols and polyols.

27. Composition according to any one of the preceding claims, **characterized in that** it also comprises at least one additive chosen from anti-hairloss agents, oxidizing agents, ceramides and pseudoceramides, vitamins and provitamins including panthenol, plant, animal, mineral or synthetic oils, waxes, sunscreens, coloured or non-coloured inorganic or organic pigments, dyes, pearlescent agents and opacifiers, sequestering agents, plasticizers, solubilizing agents, acidifying agents, basifying agents, inorganic or organic thickeners, antioxidants, hydroxy acids, fragrances and preserving agents.

28. Use of the composition according to any one of Claims 1 to 27, as a composition for the cosmetic treatment or care of the hair.

29. Use of the composition according to any one of Claims 1 to 27, as a shampoo or a composition to be applied before or after a shampoo.

30. Cosmetic treatment process, **characterized in that** it comprises the application to the hair of an effective amount of a composition according to any one of Claims 1 to 27.

## Patentansprüche

1. Kosmetische Zusammensetzung, enthaltend in einem wässerigen Milieu wenigstens ein Anti-Schuppen-Mittel, das von 2-Pyridonderivaten verschieden ist, und wenigstens ein Monolaurat von Oxyethylensorbitan, das 2 bis 10 Oxyethyleneinheiten enthält.

2. Zusammensetzung nach dem vorstehenden Anspruch, **dadurch gekennzeichnet, dass** das Anti-Schuppen-Mittel ausgewählt ist unter:
a) Pyridinethionsalzen, insbesondere Salzen von Kalzium, Magnesium, Barium, Strontium, Zink, Kadmium, Zinn und Zirkonium,
b) Trihalogencarbamiden,
c) Triclosan,
d) Azolverbindungen, wie Climbazol, Ketoconazol, Clotrinazol, Econazol, Isoconazol und Miconazol,
e) Antipilz-Polymeren, wie Amphoterizin B oder Nystatin,
f) Schwefelverbindungen von Selen, insbesondere die der Formel SₓSe_{B-x}, wobei x zwischen 1 und 7 liegt,
g) Schwefel in seinen verschiedenen Formen, Schwefel von Kadmium, Allantoin, Teere von Steinkohle oder von Holz und ihre Derivate, Salicylsäure, Undecylensäure, Fumarsäure, Allylamine, wie Terbinafin.

3. Zusammensetzung gemäß dem vorhergehenden Anspruch, **dadurch gekennzeichnet, dass** das Anti-Schuppen-Mittel ausgewählt ist unter Pyrithion von Zink, Salicylsäure, Selendisulfid und ihre Mischungen.

4. Zusammensetzung nach einem der vorhergehenden Ansprüche, **dadurch gekennzeichnet, dass** sie 0,001 bis 10 Gew.-% an Anti-Schuppen-Mittel, bezogen auf das Gesamtgewicht der Zusammensetzung, enthält.

5. Zusammensetzung nach dem vorhergehenden Anspruch, **dadurch gekennzeichnet, dass** sie 0,1 bis 5 Gew.-%, vorzugsweise 0,2 bis 2 Gew.-% Anti-Schuppen-Mittel, bezogen auf das Gesamtgewicht der Zusammensetzung, enthält.

6. Zusammensetzung nach Anspruch 1, **dadurch gekennzeichnet, dass** sie Monolaurat von Oxyethylensorbitan mit 4 Oxyethyleneinheiten enthält.

7. Zusammensetzung nach einem der vorhergehenden Ansprüche, **dadurch gekennzeichnet, dass** sie wenigstens 0,5 Gew.-% Monolaurat von Oxyethylensorbitan, enthaltend 2 bis 10 Oxyethyleneinheiten, bezogen auf das Gesamtgewicht der Zusammensetzung, enthält.

8. Zusammensetzung nach dem vorhergehenden Anspruch, **dadurch gekennzeichnet, dass** sie 0,5 bis 10 Gew.-%, vorzugweise 2 bis 9 Gew.-%, des genannten Monolaurat von Oxyethylensorbitan, bezogen auf das Gesamtgewicht der Zusammensetzung, enthält.

9. Zusammensetzung nach einem der vorhergehenden Ansprüche, **dadurch gekennzeichnet, dass** sie auch wenigstens einen Ether mit zwei Fettsäureketten, ausgewählt unter den Ethern mit zwei Fettsäureketten, die bei einer Temperatur in der Größenordnung von 30° C fest sind, entsprechend der allgemeinen Formel
R-O-R' (I)
in der R und R' gleich oder verschieden sind, und eine geradkettige oder verzweigte Alkyl- oder Alkenylgruppe mit 12 bis 30 Kohlenstoffatomen bedeutet, wobei R und R' so ausgewählt sind, dass die Verbindung der Formel (I) bei einer Temperatur von etwa 30° C fest ist.

10. Zusammensetzung nach dem vorhergehenden Anspruch, **dadurch gekennzeichnet, dass** der Ether mit zwei Fettsäureketten der Distearylether ist.

11. Zusammensetzung nach einem der Anspruch 9 und 10, **dadurch gekennzeichnet, dass** der Ether mit zwei Fettsäureketten in einer Menge von 0,5 Gew.-% oder mehr, bezogen auf das Gesamtgewicht der Zusammensetzung, vorliegt.

12. Zusammensetzung nach Anspruch 11, **dadurch gekennzeichnet, dass** der Ether mit zwei Fettsäureketten in einer Menge von 1 bis 5 Gew.-%, vorzugweise von 1,3 bis 2 Gew.-%, bezogen auf das Gesamtgewicht der Zusammensetzung, enthalten ist.

13. Zusammensetzung nach einem der vorhergehenden Ansprüche, **dadurch gekennzeichnet, dass** sie überdies wenigstens einen Fettalkohol, enthaltend wenigstens 12 Kohlenstoffatome, enthält.

14. Zusammensetzung nach Anspruch 13, **dadurch gekennzeichnet, dass** der Fettalkohol 12 bis 30 Kohlenstoffatome enthält.

15. Zusammensetzung nach Anspruch 13 oder 14, **dadurch gekennzeichnet, dass** der Fettalkohol ausgewählt ist unter Cetylalkohol, Stearylalkohol, Arachidylalkohol, Behenylalkohol, Lignocerylalkohol, Cerylalkohol und Montanylalkohol und ihren Mischungen.

16. Zusammensetzung nach einem der Ansprüche 13 bis 15, **dadurch gekennzeichnet, dass** der Fettalkohol in einer Menge von gleich 0,5 Gew.-% oder mehr, bezogen auf das Gesamtgewicht der Zusammensetzung, vorliegt.

17. Zusammensetzung nach Anspruch 16, **dadurch gekennzeichnet, dass** der Fettalkohol in einer Menge von 0,5 bis 10 Gew.-% und vorzugsweise 1 bis 5 Gew.-%, bezogen auf das Gesamtgewicht der Zusammensetzung, vorliegt.

18. Zusammensetzung nach einem der vorhergehenden Ansprüche, **dadurch gekennzeichnet, dass** sie überdies wenigstens einen anionischen, kationischen, amphoteren oder nicht-ionischen oberflächenaktiven Wirkstoff, der von Monolaurat von Oxyethylensorbitan verschieden ist und 2 bis 10 Oxyethyleneinheiten enthält, enthält.

19. Zusammensetzung nach Anspruch 18, **dadurch gekennzeichnet, dass** sie wenigstens einen anionischen und wenigstens einen amphoteren oder zwitterionischen oberflächenaktiven Wirkstoff enthält.

20. Zusammensetzung nach den Ansprüchen 18 oder 19, **dadurch gekennzeichnet, dass** der anionische oberflächenaktive Wirkstoff ausgewählt ist unter Alkylsulfaten, Alkylethersulfaten und ihren Mischungen.

21. Zusammensetzung nach einem der Ansprüche 18 bis 20, **dadurch gekennzeichnet, dass** sie von 0,1 bis 50 Gew.-% anionische aktive Wirkstoffe oder anionischen aktiven Wirkstoff, vorzugsweise von 4 bis 20 Gew.-%, bezogen auf das Gesamtgewicht der Zusammensetzung, enthält.

22. Zusammensetzung nach einem der Ansprüche 18 oder 19, **dadurch gekennzeichnet, dass** der amphotere oder zwitterionische oberflächenaktive Wirkstoff ausgewählt ist unter in (C₈₋₂₀ Alkyl)-Betainen, (C₈₋₂₀ Alkyl)-Amido(C₆₋₈ Alkyl)-Betainen und ihren Mischungen.

23. Zusammensetzung nach einem der vorhergehenden Ansprüche, **dadurch gekennzeichnet, dass** sie überdies wenigstens ein kationisches Polymer enthält.

24. Zusammensetzung nach einem der vorhergehenden Ansprüche, **dadurch gekennzeichnet, dass** sie überdies wenigstens ein Silikon enthält.

25. Zusammensetzung nach einem der vorhergehenden Ansprüche, **dadurch gekennzeichnet, dass** das wässerige Milieu von Wasser oder einer Mischung von Wasser und wenigstens einem kosmetisch annehmbaren Lösungsmittel gebildet ist.

26. Zusammensetzung nach Anspruch 25, **dadurch gekennzeichnet, dass** das Lösungsmittel ausgewählt ist unter niedrigen C₁-C₄-Alkoholen und Polyolen.

27. Zusammensetzung nach einem der vorhergehenden Ansprüche, **dadurch gekennzeichnet, dass** sie überdies einen Zusatzstoff enthält, ausgewählt unter Mitteln gegen Haarausfall, Oxydationsmitteln, Ceramiden und Pseudoceramiden, Vitaminen und Provitaminen, darunter Panthenol, pflanzliche Ölen, tierischen Ölen, Mineralölen oder synthetischen Ölen, Wachsen, Ceramiden und Pseudoceramiden, Sonnenfiltern, anorganischen oder organischen Pigmenten, Farbstoffen oder Nichtfarbstoffen, Färbemitteln, Glanzmitteln und opak machenden Mitteln, Sequestriermitteln, Plastifiziermitteln, Solubilisiermitteln, Säuerungsmitteln, Alkalisierungsmitteln, anorganischen oder organischen Verdickungsmitteln, Antioxidantien, Hydroxysäuren, Parfüms und Konservierungsmitteln.

28. Verwendung der Zusammensetzung gemäß einem der Ansprüche 1 bis 27 als Zusammensetzung zum Behandeln oder zur kosmetischen Pflege von Haaren.

29. Verwendung der Zusammensetzung nach einem der Ansprüche 1 bis 27 als Shampoo oder als Zusammensetzung zum Auftragen vor oder nach dem Shampoonieren.

30. Verfahren zum kosmetischen Behandeln, **dadurch gekennzeichnet, dass** es die Anwendung einer wirksamen Menge einer Zusammensetzung gemäß einem der Ansprüche 1 bis 27 auf Haare umfasst.
